# EUROPEAN PATENT SPECIFICATION

(11) **EP 4 643 861 B1**
(45) Date of publication and mention of the grant of the patent: **29.07.2026**
(21) Application number: 24173602.4
(22) Date of filing: 30.04.2024
(51) Int. Cl.: A61K 31/506, A61K 45/06, A61P 35/00, A61P 35/04

(54) **A HALOGENATED HETEROARYL KINASE INHIBITOR FOR USE IN TREATING ANGIOSARCOMA**
HALOGENIERTER HETEROARYLKINASEHEMMER ZUR VERWENDUNG BEI DER BEHANDLUNG VON ANGIOSARKOM
INHIBITEUR DE KINASE HETEROARYLE HALOGENEE POUR UTILISATION DANS LE TRAITEMENT DE L'ANGIOSARCOME

(43) Date of publication of application: 05.11.2025
(73) Proprietor: iOmx Therapeutics AG, 82152 Martinsried (DE)
(72) Inventor: LOFERER, Hannes, 82152 Martinsried (DE); YULE, Murray, 82152 Martinsried (DE); MASER, Petra, 82152 Martinsried (DE); STEBEGG, Marisa, 82152 Martinsried (DE); BISSINGER, Stefan, 82152 Martinsried (DE); BONI, Valentina, 28223 Pozuelo de Alarcón, Madrid (ES); FUENTES ANTRÁS, Jesús, 28223 Pozuelo de Alarcón, Madrid (ES)
(74) Representative: Kuttenkeuler, David

(56) References cited:
- WO-A1-2021/214117
- WO-A1-2023/194622
- LEWCUN JOSEPH A ET AL: "Doxorubicin, paclitaxel, and cisplatin based chemotherapy for the treatment of angiosarcoma: Two case reports", INTERNATIONAL JOURNAL OF SURGERY CASE REPORTS, ELSEVIER, AMSTERDAM, NL, vol. 68, 1 January 2020 (2020-01-01), pages 83 - 87, XP086127228, ISSN: 2210-2612, [retrieved on 20200221], DOI: 10.1016/J.IJSCR.2020.02.036

## Description

The present invention relates to pharmaceutical composition for use in a treatment of angiosarcoma, comprising a halogenated heterocyclic kinase inhibitor as defined in the claims.

Soft tissue sarcomas (STS) are a heterogeneous group of rare cancers that arise from the soft tissues of the body, such as muscles, fat, nerves, blood vessels, and other connective tissues. These tumors can occur anywhere in the body but are most commonly found in the arms, legs, and abdomen. Due to their rarity and diversity, diagnosing and treating soft tissue sarcomas presents unique challenges and necessitates a multidisciplinary approach. This introduction delves into the current state of diagnosis and treatment strategies for soft tissue sarcoma, highlighting the latest advancements and ongoing challenges in the field. Treatment options of soft tissue sarcomas are still unsatisfactory todate. The cornerstone of treatment for localized sarcomas is surgical resection with the aim of removing the tumor entirely while preserving as much function as possible. Further often used as treatment option of soft tissue sarcomas is radiotherapy, for example preoperatively to shrink tumors and make them easier to remove or postoperatively to kill any remaining cancer cells. Also chemotherapy such as with paclitaxel is used in metasatatic cases to treat sarcoma patients. However, effectiveness of chemotherapy varies widely among different sarcoma subtypes.

Angiosarcoma is a rare and aggressive form of soft tissue sarcoma that originates in the cells that line blood vessels or lymph vessels. Due to its high grade and tendency to metastasize, treating angiosarcoma can be challenging. Recently, targeted therapies have emerged as a promising approach to manage this disease, focusing on specific molecular and genetic features of the tumor to improve outcomes. In particular the use of anti-angiogenic compounds has emerged as possible treatment strategy. In particular anti-VEGF agents should be mentioned, and most importantly the compound called pazopanib. Pazopanib (Votrient) is an oral, multitargeted, tyrosine kinase inhibitor (TKI) that was originally approved by the US Food and Drug Administration (FDA) in 2009 for the treatment of advanced renal cell carcinoma (RCC). In April 2012 it gained approval for the treatment of advanced soft tissue sarcoma (STS) in patients who have received prior chemotherapy [J Adv Pract Oncol. 2013 Jan-Feb; 4(1): 53-57].

Other than pazopanib, target therapies under development further include the use other anti-VEGF agents such as sorafenib, immune therapeies including therapeutics directed at the PD1-PDL1 axis, beta blocker treatments, and with antibodies such as olaratumab which is a monoclonal antibody that targets the platelet-derived growth factor receptor alpha (PDGFR-a), which is a tyrosine kinase receptor involved in cell growth and angiogenesis.

Protein tyrosine kinases have been identified as crucial targets in the therapeutic treatment of cancer. Growth factor ligands and their respective receptor tyrosine kinases are required for tumor angiogenesis and growth. Vascular endothelial growth factor (VEGF) is a critical component in the process leading to the branching, extension, and survival of endothelial cells forming new blood vessels during angiogenesis. Unwanted angiogenesis is a hallmark of several diseases, such as retinopathies, psoriasis, rheumatoid arthritis, age-related macular degeneration (AMD), and cancer (including solid tumors) Folkman, Nature Med., 1, 27-31 (1995). VEGF is a dimeric, disulfide-linked 46-kDa glycoprotein produced by normal cell lines and tumor cell lines. It is an endothelial cell-specific mitogen, shows angiogenic activity in in vivo test systems, e. g. , rabbit cornea, is chemotactic for endothelial cells and monocytes, and induces plasminogen activators in endothelial cells, which are then involved in the proteolytic degradation of extracellular matrix during the formation of capillaries. A number of isoforms of VEGF are known, which show comparable biological activity, but differ in the type of cells that secrete them and in their heparin-binding capacity. In addition, there are other members of the VEGF family, such as placenta growth factor and VEGF-C. VEGF receptors are transmembranous receptor tyrosine kinases. They are characterized by an extracellular domain with seven immunoglobulinlike domains and an intracellular tyrosine kinase domain. Various types of VEGF receptor are known, e. g., VEGFR-1, VEGFR-2 and VEGFR-3.

SIK3 is an intracellular serine/threonine kinase belonging to the AMPK superfamily. Salt-inducible kinases (SIKs) constitute a serine tyrosine kinase subfamily, belonging to the adenosine monophosphate-activated kinase (AMPK) family. Three members (SIK1, -2, and -3) have been identified so far. Amino acid homology of SIK1 with SIK2 and SIK3 is 78% and 68%, respectively, in the kinase domain. The cloning of SIK1 (also known as SIK and SNF1LK), abundantly expressed in the adrenal glands of high-salt, diet-fed rats, led to subsequent cloning of SIK2 (also known as QIK, KIAA0781 and SNF1LK2), mainly expressed in adipose tissues and the rather ubiquitous SIK3 (also known as QSK, KIAA0999 or L19) (Katoh et al. 2004, Mol. Cell. Endocrinol. 217:109). The three SIKs have a similar structure, with an N-terminal kinase domain (catalytic domain), a middle ubiquitin-associated domain (believed important for phosphorylation by LKB1) and a long C-terminal sequence (believed to be a site for further phosphorylation by PKA). However, there are very diverse roles implicated for the various SIKs. For example, various SIKs have been implicated in biological processes as diverse as osteocyte response to parathyroid hormone (Wein et al. 2016, Nature Commun. 7:13176) to induction of SIK1 by gastrin and inhibition of migration of gastric adenocarcinoma cells (Selvik et al. 2014, PLoS ONE 9:ell2485). Other potential roles of salt-inducible kinases (in particular SIK3), described in WO2018/193084A1 (to the present applicant, and published 25-Oct-2018), are furthermore that SIK3 is involved in tumour cell resistance to cell-mediated immune responses, in particular tumour cell resistance to TNF.

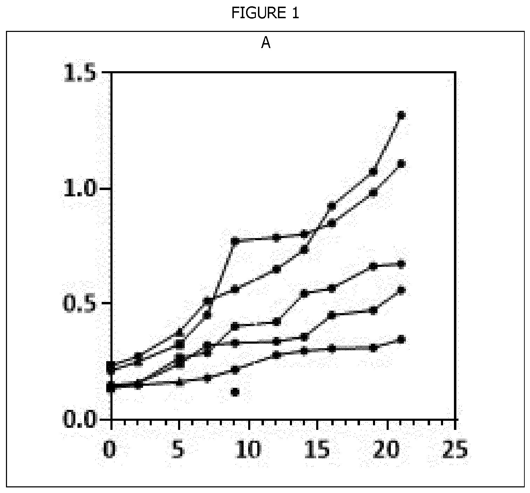

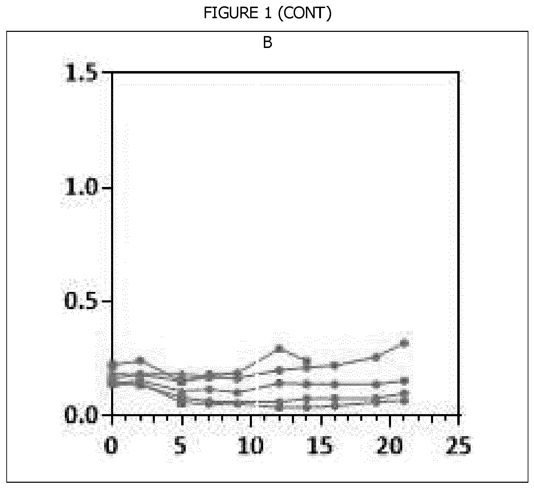

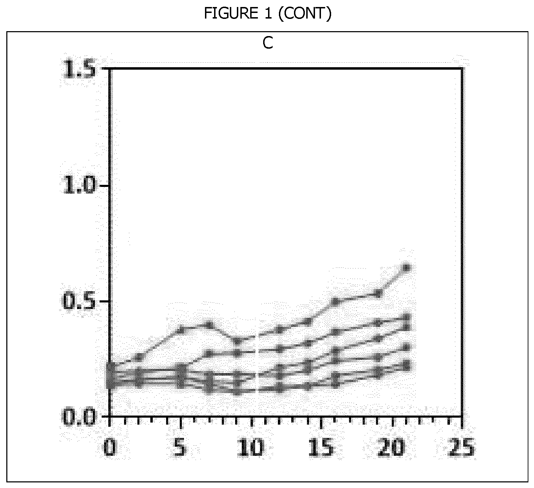

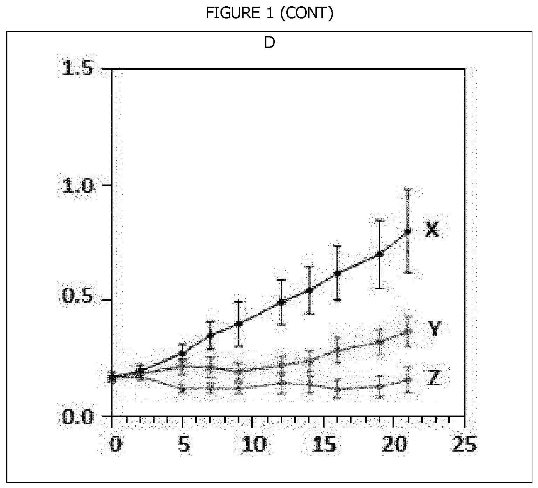

The applicants recently reported (WO2020/083926; Michels et al, 2020; Cancer Res 80(AACR Suppl 16):Abstract 6698) on the development of a first-in-class, potent, oral inhibitor of SIK3 described as "C7" (Figure 1). Compound C7 effectively engages the SIK3 pathway in tumour cells by inhibiting the phosphorylation of its substrate HDAC4 and abrogating the TNF-induced transcriptional activity of NFKB in a dose-dependent manner both in vitro and in vivo. As a result, C7 makes tumour cells, both human and murine, increasingly sensitive to TNF-mediated lysis while sparing healthy PBMCs in vitro. Treatment of established tumours in different syngeneic tumour mouse models (MC38, EMT6) with C7 resulted in significant tumour growth inhibition in monotherapy setting. The effect was comparable or even superior to anti-PD-1 treatment alone. Furthermore, immune cell profiling of treated mice showed a significant infiltration of activated T cells, along with remarkable reduction in immunosuppressive Tregs and M2 TAMs. Given the emerging role of TNF resistance in tumour immune evasion and known dependency on NFKB pathway by multiple solid tumours, SIK3 inhibitors that work by abrogating TNF-driven NFKB activity merit further investigation in the clinics for the treatment of cancer.

International patent publication WO2021/214117 concerns kinase inhibitors, in particular inhibitors of protein kinases that were developed as specific and selective inhibitors of the SIK3 kinase. Although structurally similar to dasatinib, the kinase inhibitors of WO2021/214117 are distinctive; possessing a particular class of halogenated heteroaryls. As is described by the present applicant in WO2021/214117, a superior SIK3 inhibiting compound named "E9" (Figure 1) was found to possess surprisingly improved drug-like properties over the structurally-similar thiophene compound C7, as well as over other structurally-related compounds D9, B3 and A8 (Figure 1).

WO 2021/214117 discloses halogenated-heteroaryl and other heterocyclic kinase inhibitors.

Lewcun, Joseph A., et al. ( in "Doxorubicin, paclitaxel, and cisplatin based chemotherapy for the treatment of angiosarcoma: two case reports." International Journal of Surgery Case Reports 68 (2020): 83-87") discloses case reports of chemotherapy for treating angiosarcoma.

Current methods of treatment of soft tissue sarcomas and in particular of angiosarcomas are still insufficient. Surgery removes the diseased tissue; radiotherapy shrinks solid tumors; and chemotherapy kills rapidly dividing cells. However, resection often does not sufficiently remove canerous tissue, and chemotherapy, in particular, results in numerous side effects, in some cases so severe as to limit the dosage that can be given and thus preclude the use of potentially effective drugs. Moreover, cancers often develop resistance to chemotherapeutic drugs. Thus, there is an urgent need for specific and more effective targeted therapies of soft tissue sarcoma including angiosarcoma.

### SUMMARY OF THE INVENTION

The above problem is solved by the various aspect indicated herein below. The invention is predicated upon the surprising finding that a kinase inhibitor -(2-chloro-4-(fluoromethyl)thiophen-3-yl)-2-((2-methyl-6-(4-methylpiperazin-1-yl)pyrimidin-4-yl)amino)thiazole-5-carboxamide) - herein referred to as compound E9 - provides excellent treatment outcomes with unusualy long progression free survival (PFS) as well as an unusual extended treatment response time when administered to subject suffering from angiosarcoma. While E9 has been developed as a SIK3 kinase inhibitor, the herein described therapeutic profile of E9 in angiosarcoma xenograft *in-vivo* models and in a treated angiosarcoma human, was unexpected and provides a new targeted treatment option to tackle soft tissue sarcoma.

Generally, and by way of brief description, the main aspects of the present invention can be summarised as follows:

In a **first aspect** the invention provides a **pharmaceutical** composition for use in a treatment of angiosarcoma in a subject, wherein the pharmaceutical composition comprises a kinase inhibitor according to formula (I), herein also referred to as E9, 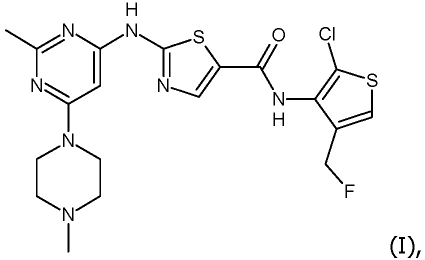 (*N*-(2-chloro-4-(fluoromethyl)thiophen-3-yl)-2-((2-methyl-6-(4-methylpiperazin-1-yl)pyrimidin-4-yl)amino)thiazole-5-carboxamide)
Or solvates or salts thereof; together with a pharmaceutically acceptable carrier, diluent and/or excipient. (i)

### BRIEF DESCRIPTION OF THE FIGURES

The figures show:
**Figure 1****:** depicts the E9 monotherapy efficacy in a Human Patient-Derived Xenograft (PDX) Angiosarcoma model in Immuno-deficient NOD scid gamma (NOG) mice; tumor growth of single mice treated either with **(A)** vehicle control, with **(B)** 50mg/kg E9 or with **(C)** 25mg/kg E9; Figure **1D** depicts the mean tumor growth inhibition of all three groups (mean +/- SEM), with X = vehicle control, Y = 25mg/kg E9, Z = 50mg/kg E9. X-axis: Days after treatment start; Y-axis: Tumour volume (mm³).

### DETAILS OF THE PRESENT INVENTION

The present invention, and particular aspects and/or embodiments thereof, can be described in more detail as follows.

Although the present invention may be further described in more detail, it is to be understood that this invention is not limited to the particular methodologies, protocols and reagents described herein. It is also to be understood that the terminology used herein is for the purpose of describing particular embodiments only, and is not intended to limit the scope of the present invention which will be limited only by the appended claims.

Herein, certain elements of the present invention are described in more detail. These elements are listed with specific embodiments, however, it should be understood that they may be combined in any manner and in any number to create additional embodiments. The variously described examples and preferred embodiments should not be construed to limit the present invention to only the explicitly described embodiments. This description of this application should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise. For example, if in one embodiment of the use of a compound of the invention the subject is an adult human and in another embodiment of the use of a compound of the invention the soft tissue sarcoma is angiosarcoma, then in a preferred embodiment of the use of a compound of the invention, the subject is an adult human and the soft tissue sarcoma is angiosarcoma.

### General definitions

Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The practice of the present invention will employ, unless otherwise indicated, conventional methods of chemistry, biochemistry, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The term "consisting essentially of" means excluding other members, integers or steps of any essential significance or group of members, integers or steps of any essential significance. For example, a pharmaceutical composition consisting essentially of the members/components as defined herein (such as a compound as defined in any of the aspects of the invention and optionally one additional therapeutic agent) would exclude further therapeutic agents (besides the compound as defined in any of the aspects of the invention and the optional one additional therapeutic agent) but would not exclude contaminants (e.g., those from the isolation and purification method) in trace amounts (e.g., the amount of the contaminant (preferably the amount of all contaminants present in the composition) is less than 5% by weight, such as less than 4% by weight, 3% by weight, 2% by weight, 1% by weight, 0.5% by weight, 0.4% by weight, 0.3% by weight, 0.2% by weight, 0.1% by weight, 0.05% by weight, with respect to the total composition) and/or pharmaceutically acceptable excipients (such as carriers, e.g., phosphate buffered saline, preservatives, and the like). The term "consisting of" means excluding all other members, integers or steps of significance or group of members, integers or steps of significance. For example, a pharmaceutical composition consisting of the members/components as defined herein (such as a compound as defined in any of the aspects of the invention, one excipient, and optionally one additional therapeutic agent) would exclude any other compound (including a second or further excipient) in an amount of more than 2% by weight (such as any other compound in an amount of more than 1% by weight, more than 0.5% by weight, more than 0.4% by weight, more than 0.3% by weight, more than 0.2% by weight, more than 0.1% by weight, more than 0.09% by weight, more than 0.08% by weight, more than 0.07% by weight, more than 0.06% by weight, more than 0.05% by weight, more than 0.04% by weight, more than 0.03% by weight, more than 0.02% by weight, more than 0.01% by weight) with respect to the total composition. The term "comprising" encompasses the term "consisting essentially of" which, in turn, encompasses the term "consisting of". Thus, at each occurrence in the present application, the term "comprising" may be replaced with the term "consisting essentially of" or "consisting of". Likewise, at each occurrence in the present application, the term "consisting essentially of" may be replaced with the term "consisting of".

Where used herein, "and/or" is to be taken as specific disclosure of each of the two specified features or components with or without the other. For example, "X and/or Y" is to be taken as specific disclosure of each of (i) X, (ii) Y, and (iii) X and Y, just as if each is set out individually herein.

In the context of the present invention, the terms "about" and "approximately" are used interchangeably and denote an interval of accuracy that the person of ordinary skill will understand to still ensure the technical effect of the feature in question. The term typically indicates deviation from the indicated numerical value by ±5%, ±4%, ±3%, ±2%, ±1%, ±0.9%, ±0.8%, ±0.7%, ±0.6%, ±0.5%, ±0.4%, ±0.3%, ±0.2%, ±0.1%, 0.05%, and for example ±0.01%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect.

The terms "a", "an" and "the" and similar references used in the context of describing the invention (especially in the context of the claims) are to be construed to cover both the singular and the plural, unless otherwise indicated herein or clearly contradicted by the context.

Recitation of ranges of values herein is merely intended to serve as a shorthand method of referring individually to each separate value falling within the range. Unless otherwise indicated herein, each individual value is incorporated into the specification as if it were individually recited herein.

All methods described herein can be performed in any suitable order unless otherwise indicated herein or otherwise clearly contradicted by the context.

The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Several documents are cited throughout the text of this specification. Nothing herein is to be construed as an admission that the invention is not entitled to antedate such disclosure by virtue of prior invention.

The terms "of the [present] invention", "in accordance with the [present] invention", "according to the [present] invention" and the like, as used herein are intended to refer to all aspects and embodiments of the invention claimed herein.

Unless context dictates otherwise, the descriptions and definitions of the features set out above or below are not limited to any particular aspect or embodiment of the invention and apply equally to all aspects and embodiments that are described.

"Polymorphism" as referred to herein means that a solid material (such as a compound) is able to exist in more than one form or crystalline structure, i.e., "polymorphic modifications" or "polymorphic forms". The terms "polymorphic modifications", "polymorphic forms", and "polymorphs" are used interchangeable in the present invention. According to the present invention, these "polymorphic modifications" include crystalline forms, amorphous forms, solvates, and hydrates. Mainly, the reason for the existence of different polymorphic forms lies in the use of different conditions during the crystallization process, such as the following:
- solvent effects (the packing of crystal may be different in polar and nonpolar solvents);
- certain impurities inhibiting growth pattern and favor the growth of a metastable polymorphs;
- the level of supersaturation from which material is crystallized (in which generally the higher the concentration above the solubility, the more likelihood of metastable formation);
- temperature at which crystallization is carried out;
- geometry of covalent bonds (differences leading to conformational polymorphism);
- change in stirring conditions.

Polymorphic forms may have different chemical, physical, and/or pharmacological properties, including but not limited to, melting point, X-ray crystal and diffraction pattern, chemical reactivity, solubility, dissolution rate, vapor pressure, density, hygroscopicity, flowability, stability, compactability, and bioavailability. Polymorphic forms may spontaneously convert from a metastable form (unstable form) to the stable form at a particular temperature. According to Ostwald's rule, in general it is not the most stable but the least stable polymorph that crystallizes first. Thus, quality, efficacy, safety, processability and/or manufacture of a chemical compound, such as a compound of the present invention, can be affected by polymorphism. Often, the most stable polymorph of a compound (such as a compound of the present invention) is chosen due to the minimal potential for conversion to another polymorph. However, a polymorphic form which is not the most stable polymorphic form may be chosen due to reasons other than stability, e.g. solubility, dissolution rate, and/or bioavailability.

The term "crystalline form" of a material as used herein means that the smallest components (i.e., atoms, molecule or ions) of said material form crystal structures. A "crystal structure" as referred to herein means a unique three-dimensional arrangement of atoms or molecules in a crystalline liquid or solid and is characterized by a pattern, a set of atoms arranged in a particular manner, and a lattice exhibiting long-range order and symmetry. A lattice is an array of points repeating periodically in three dimensions and patterns are located upon the points of a lattice. The subunit of the lattice is the unit cell. The lattice parameters are the lengths of the edges of a unit cell and the angles between them. The symmetry properties of the crystal are embodied in its space group. In order to describe a crystal structure the following parameters are required: chemical formula, lattice parameters, space group, the coordinates of the atoms and occupation number of the point positions.

The term "amorphous form" of a material as used herein means that the smallest components (i.e., atoms, molecule or ions) of said material are not arranged in a lattice but are arranged randomly. Thus, unlike crystals in which a short-range order (constant distances to the next neighbor atoms) and a long-range order (periodical repetition of a basic lattice) exist, only a short-range order exists in an amorphous form.

The term "complex of a compound" as used herein refers to a compound of higher order which is generated by association of the compound with one or more other molecules. Exemplary complexes of a compound include, but are not limited to, solvates, clusters, and chelates of said compound.

The term "solvate" as used herein refers to an addition complex of a dissolved material in a solvent (such as an organic solvent (e.g., an aliphatic alcohol (such as methanol, ethanol, n-propanol, isopropanol), acetone, acetonitrile, ether, and the like), water or a mixture of two or more of these liquids), wherein the addition complex exists in the form of a crystal or mixed crystal. The amount of solvent contained in the addition complex may be stoichiometric or nonstoichiometric. A "hydrate" is a solvate wherein the solvent is water.

In isotopically labeled compounds one or more atoms are replaced by a corresponding atom having the same number of protons but differing in the number of neutrons. For example, a hydrogen atom may be replaced by a deuterium atom. Exemplary isotopes which can be used in the compounds of the present invention include deuterium, ¹¹C, ¹³C, ¹⁴C, ¹⁵N, ¹⁸F, ³²P, ³²S, ³⁵S, ³⁶Cl, and ¹²⁵I.

The expression "amino protecting group" as used herein preferably refers to any group by which an amino group contained in a compound can be transferred into a less reactive (i.e., protected) amino group. Preferably, amino protecting groups can be incorporated into the corresponding compound under mild conditions, in a chemoselective and/or regioselective manner, and/or in good yields. Furthermore, the amino protecting groups should be stable under the conditions to which the protected compound is to be subjected (e.g., the conditions of the desired reaction and/or purification conditions). Preferably, the amino protecting groups should minimize the risk of racemization of a stereogenic center, when present in the compound. In one embodiment, the amino protecting groups should be removable from the protected compound under mild conditions and in a selective manner such that the deprotected compound is obtained in high yields. Exemplary amino protecting groups include tert-butyloxycarbonyl (BOC), 9-fluorenylmethoxycarbonyl (FMOC), benzyloxycarbonyl (Cbz), p-methoxybenzylcarbonyl (MOZ), acetyl (Ac), trifluoroacetyl, benzoyl (Bz), benzyl (Bn), p-methoxybenzyl (PMB), 3,4-dimethoxyphenyl (DMPM), p-methoxyphenyl (PMP), 2,2,2-trichloroethoxycarbonyl (Troc), triphenylmethyl (trityl; Tr), toluenesulfonyl (tosyl; Ts), para-bromophenylsulfonyl (brosyl), 4-nitrobenzenesulfonyl (nosyl), and 2-nitrophenylsulfenyl (Nps).

The term "half-life" relates to the period of time which is needed to eliminate half of the activity, amount, or number of molecules. In the context of the present invention, the half-life of a compound disclosed herein (eg a compound of formula (Ia), (Ib) or (Ic)) is indicative for the stability of said compound.

The terms "subject", "patient", "individual", or "animal" relate to multicellular animals, such as vertebrates. For example, vertebrates in the context of the present invention are mammals, birds (e.g., poultry), reptiles, amphibians, bony fishes, and cartilaginous fishes, in particular domesticated animals of any of the foregoing as well as animals (in particular vertebrates) in captivity such as animals (in particular vertebrates) of zoos. Mammals in the context of the present invention include, but are not limited to, humans, non-human primates, domesticated mammals, such as dogs, cats, sheep, cattle, goats, pigs, horses etc., laboratory mammals such as mice, rats, rabbits, guinea pigs, etc. as well as mammals in captivity such as mammals of zoos. The term "animal" as used herein also includes humans. Particular non-limiting examples of birds include domesticated poultry, and include birds such as chickens, turkeys, ducks, geese, guinea fowl, pigeons, pheasants etc.; while particular non-limiting examples of bony or cartilaginous fish include those suitable for cultivation by aquiculture, and include bony fish such as salmon, trout, perch, carp, cat-fish, etc.

As used herein, the terms "effective response" of a patient or a patient's "responsiveness" to treatment with of the agents, or "therapeutic effect" refers to the clinical or therapeutic benefit(s) imparted to a patient upon administration. As used herein, an "unexpected therapeutic effect" of the treatment of the invention is the ability to produce marked anti-cancer effects in a patient without causing significant toxicities or adverse effects, so that the patient benefits from the treatment overall. The efficacy, i.e., therapeutic effect(s), of the treatment of the invention can be measured by various endpoints commonly used in evaluating cancer treatments, include any one or more including, but not limited to: extending survival (including OS and PFS); resulting in an objective response (including a CR or a PR); tumor regression, tumor weight or size shrinkage, longer time to disease progression, increased duration of survival, longer PFS, improved OS rate, increased duration of response, and improved quality of life and/or improving signs or symptoms of cancer, etc. Because the invention relates to the use of a combination of unique anti-tumor agents, novel approaches to determine efficacy, i.e., therapeutic effect(s), of any particular combination therapy of the present invention can be optionally employed, including, for example, measurement of plasma or urinary markers of angiogenesis and measurement of response through radiological imaging.

As used herein, the term "disease progression" or "progressive disease" (PD), used interchangeably herein, refers to least a 20% increase in the sum of diameters of target lesions, taking as reference the smallest sum on study (this includes the baseline sum if that is the smallest on study). In addition to the relative increase of 20%, the sum must also demonstrate an absolute increase of at least 5 mm. The appearance of one or more new lesions is also considered progression.

As used herein, the term "partial response," (PR) refers to least a 30% decrease in the sum of diameters of target lesions, taking as reference the baseline sum diameters.

As used herein, the term, "complete response" (CR) refers to the disappearance of all target lesions. Any pathological lymph nodes (whether target or non-target) must have reduction in short axis to < 10 mm. As used herein, the term "stable disease" (SD) refers to a tumor diameter that does not shrink sufficiently to qualify for PR or sufficient increase to qualify for PD, taking as reference the smallest sum diameters while on Study.

As used herein, the term "objective response" (OR) refers to a measurable response, including CR or PR.

As used herein, the term, "overall survival" (OS) refers to the patient remaining alive for a defined period of time, such as 1 year, 5 years, etc. from the time of diagnosis or treatment. In a preferred aspect of the invention, for the Study, overall survival is defined as the time from the date of randomization in the Study to the date of death from any cause; if the patient is alive at the end of the follow-up period or is lost to follow-up, OS will be censored on the last date the patient is known to be alive.

As used herein, the term, "progression-free survival" (PFS) refers to the patient remaining alive without the cancer progressing or getting worse.

As used herein, the term "extending survival" or "prolonged survival" which are used interchangeably herein, refers to increasing OS or PFS in a treated patient relative to i) an untreated patient, ii) a patient treated with less than all of the anti-tumor agents in a particular combination therapy, or iii) a control treatment protocol.

In a **first aspect** the invention provides a **pharmaceutical** composition for use in a treatment of soft tissue sarcoma in a subject, wherein the soft tissue sarcoma is an angiosarcoma, and wherein the composition is for enteral or parenteral administration to the subject and comprises a kinase inhibitor according to formula (I), herein also referred to as E9, 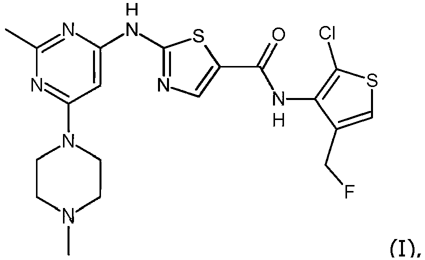 (*N-*(2-chloro-4-(fluoromethyl)thiophen-3-yl)-2-((2-methyl-6-(4-methylpiperazin-1-yl)pyrimidin-4-yl)amino)thiazole-5-carboxamide)
or solvates or salts thereof; together with a pharmaceutically acceptable carrier, diluent and/or excipient.

In a preferred embodiment of the invention, the soft tissue sarcoma is characterized by the expression of Salt Induced Kinase 3 (SIK3) in cells associated with the soft tissue sarcoma, preferably in soft tissue sarcoma cells. In an alternative embodiment, however, the soft tissue sarcoma is independent of SIK3 expression, or, in certain specific alternative embodiments, the soft tissue sarcoma is characterized by not expressing Salt Induced Kinase 3 (SIK3) in cells associated with the soft tissue sarcoma, preferably in the soft tissue sarcoma cells.

The invention pertains to embodiments, wherein the soft tissue sarcoma is angiosarcoma. In embodiments, the angiosarcoma is for example characterized by the occurrence of cutaneous lesions.

The invention may include embodiments wherein the soft tissue sarcoma is either a primary or is a secondary angiosarcoma, in particular a secondary angiosarcoma. The present invention may include a variety of aetiologies associated with the pathology of the angiosarcoma. For example, the angiosarcoma may have occurred in context of radiation, chronic lymphoedema, environmental carcinogens or the patient may have a history genetic syndromes. In particular, radiotherapy induced angiosarcoma due to treatment of a primary breast tumor is a major subgroup of secondary angiosarcoma.

Therefore, the present invention preferably pertains to embodiments wherein the subject to be treated suffers from angiosarcoma and the subject is characterized by a prior treatment history of radiotherapy for a different (primary) disease, for example radiotherapy for the treatment of breast cancer.

In context of the invention, the soft tissue sarcoma treatable with the therpies disclosed herein, may be at any stage. However, in particular treatment of soft tissue sarcoma is contemplated wherein the cancer is metastatic or unresectable. Late stage metastatic soft tissue sarcoma, such as a metastatic angiosarcoma, may include an additional chemotherapeutic treaetment, such as with a taxane (paclitaxel) or doxorubicin. Combination therpies are described herein elsewhere.

The invention preferably pertains to embodiments wherein the kinase inhibitor is administered orally.

The invention preferably pertains to embodiments wherein the kinase inhibitor is formulated as a solid dosage form such as a capsule or tablet.

The invention preferably pertains to embodiments wherein the kinase inhibitor is formulated as a single dosage form comprising in one such single dosage form 20mg to 100mg of the kinase inhibitor.

The invention preferably pertains to embodiments wherein the kinase inhibitor is administered every 12 hours.

The invention preferably pertains to embodiments wherein the treatment is continued during and after remission of the tumor.

The invention in some embodiments pertains to a treatment wherein the administration of the compound (E9) results in prolonged survival and/or continuous treatment response, preferably of at least 3 months, more preferably at least 4 months, more preferably at least 5 months, more preferably at least 6 months, more preferably at least 7 months, more preferably at least 8 months, more preferably at least 10 months, or longer.

The invention pertains to embodiments wherein the treatment comprises an administration of at least one additional therapeutic to the subject. Additional therapeutic treatment options include classical therapy options such as radiotherapy, surgical removal of the tumor (or combinations of the two). Further included is the administration of futher therapeutic agents or further therapeutic combinations, known to be effective for the treatment of the soft tissue sarcoma.

In preferred embodiments the additional therapeutic is selected from an immune therapy, such as an anti-PD1/PDL1 therapeutic, a taxane chemotherapeutic, such as paclitaxel, an anthracycline such as doxorubicin or liposomal doxorubicin, a PDGF/PDGFR inhibitor, such as olaratumab, a VEGF/VEGFR inhibitor, such as pazopanib or sorafenib, and β-blockers such as propranolol.

As used herein the term "VEGFR inhibitor" or "VEGFR (signalling) inhibitor" relates to a compound or composition that is known to anatagonize one or more of the biological functions of VEGF. The term "VEGF" refers to a vascular endothelial growth factor that induces angiogenesis or an angiogenic process. As used herein, the term "VEGF" may include the various subtypes of VEGF. Further, as used herein, the term "VEGF" may include VEGF-related angiogenic factors such as PIGF (placenta growth factor), VEGF-B, VEGF-C, VEGF-D and VEGF-E, which act through a cognate VEFG receptor (e.g., VEGFR) to induce angiogenesis or an angiogenic process. The term "VEGF" may include any member of the class of growth factors that binds to a VEGF receptor such as VEGFR-1 (Flt-I), VEGFR-2 (KDR/Flk-I), or VEGFR-3 (FLT-4). The term "VEGF" can be used to refer to a "VEGF" polypeptide or a "VEGF" encoding gene or nucleic acid.

The term "VEGF antagonist" refers to an agent that reduces, or inhibits, either partially or fully, the activity or production of a VEGF. In certain embodiments, the VEGF antagonist inhibits one or more of VEGF-A, VEGF-B, VEGF-C and VEGF-D. A VEGF antagonist can directly or indirectly reduce or inhibit the activity or production of a specific VEGF such as VEGFi65. Furthermore, "VEGF antagonists" may include agents that act on either a VEGF ligand or its cognate receptor so as to reduce or inhibit a VEGF-associated receptor signal. Examples of "VEGF antagonists" may include antisense molecules, ribozymes or RNAi that target a VEGF nucleic acid; anti-VEGF aptamers, anti-VEGF antibodies to VEGF itself or its receptor, or soluble VEGF receptor decoys that prevent binding of a VEGF to its cognate receptor; antisense molecules, ribozymes, or RNAi that target a cognate VEGF receptor (VEGFR) nucleic acid; anti-VEGFR aptamers or anti-VEGFR antibodies that bind to a cognate VEGFR receptor; and VEGFR tyrosine kinase inhibitors. In certain embodiments, the VEGF antagonist is a peptide, e.g., a peptide comprising three or more amino acid residues. In certain embodiments, the VEGF antagonist is a bicyclic peptide. In certain embodiments, the VEGF antagonist is a gene therapy, vector, or delivery vehicle delivering a plasmid, gene, or other genetic sequence capable of giving rise to a moiety that antagonizes VEGF

Preferably, in the various embodiments of the aspects of the invention, the term VEGFR-signalling inhibitor is a compound known for its activity to antagonize one or more of VEGFR1, VEGFR2 and VEGFR3. The person of skill knows that there are various compounds with varying structural composition but that have such desired VEGFR inhibitory activity. Without being restricted to any specific compound, the invention preferably relates to an VEGFR signalling inhibitor which is selected from the group consisting of axitinib, ramicurimab, bevacuzamab, ranizumab, aflibercept, HLX12, ziv-aflibercept, vanucizumab, TX16, UB-922, BEVZ92, BCD-021, BI695502, CHS-5217, JHL1149, FKB238, Abevmy, ONS1045, PF06439535, HD204, SB8, TAB008, RPH001, BP102, HLX04, CT-P16, IBI305, LY01008, Mvasi, apagin, ranibizumab, CHS-3351, PF582, Xlucane, FYB201, razumab, CHS-2020, FYB203, ABP- 201, sevacizumab, brolucizumab, CSL346, faricimab, hPV19, TAB014, UB-924, VGX-100, VX70, STI- A0168, CVX-241, BI 836880, ABT-165, conbercept, MP0250, MP0260, angiocal, abicipar pegol, anlotinib, apatinib, altiratinib, vandetanib, linifanib, motesanib, necuparanib, HLX12, APX004, CDP791, HLX-06, IBI302, icrucumab, IMC-1C11, IMC-3C5, MSB0254, navicixizumab, tanibirumab, V-DOS47, cabozantib, brivanib, dovitinib lactate, famitinib, foretinib, fruquintinib, golvatinib, henatinib, ponatinib, lenvatinib, lucitanib, sorafenib, nintedanib, orantinib, pegdinetanib, cediranib, rivoceranib, midostaurin, sitravatinib, regorafenib, sunitinib, sulfatnib, tesevatinib, tivozanib, valatanib, or pazopanib. In preferred embodiments of the invention the VEGFR signalling inhibitor is pazopanib or axitinib.

As used herein the term "PD1 inhibitor" refers to an agent which interferes with PD1 activation or function. Examples of PD1 inhibitors include PD1 antibodies (e.g. PD1, PDL1 or PDL2 antibodies); small organic molecule PD1 antagonists; and/or agents that bind to, or interfere with function of PD1. Typically, the PD1 inhibitor is an antibody or small organic molecule which binds to the PD1 receptor or to its ligand PDL1 or PDL2.

In some embodiments, the PD1 inhibitor is a small organic molecule. As used herein, the term "small organic molecule" refers to a molecule of size comparable to those organic molecules generally used in pharmaceuticals. The term excludes biological macromolecules (e.g.; proteins, nucleic acids, etc.); preferred small organic molecules range in size up to 2000 Da, and most preferably up to about 1000 Da.

A non-exhaustive list of PD1/PDL1 antibodies comprises: Pembrolizumab (Keytruda^{®}), Nivolumab (Opdivo^{®}), BMS-936559 (MDX 1105), Cemiplimab (REGN2810), Cemiplimab-rwlc (LIBTAYO^{®}), Avelumab (MSB0010718C or Bavencio), Durvalumab (MEDI4736 or INFIMZI^{®}), Atezolizumab (MPDL3280A or Tecentriq^{®}), Spartalizumab (PDR 001), as well as their combination.

The invention preferably pertains to embodiments, wherein the angiosarcoma is resistant to a standard of care chemotherapy, preferably resistant to a treatment with a chemotherapeutic selected from a taxene, such as paclitaxel, doxorubicin and/or cyclophosphamide.

### Kinase Inhibitor Compound of the Invention (Compound E9)

"Salt-inducible kinase 3" or "SIK3" (synonyms QSK and KIAA0999) is a member of a subfamily of serine/threonine protein kinases including SIK1, SIK2, and SIK3 that belong to an AMP-activated protein kinase (AMPK) family. A SIK3 protein in context of the invention is, typically, a protein kinase. Pertinent information on the human SIK3 protein is accessible on UniProt: Q9Y2K2 (Entry version 138 of 15-Mar-2017) and a SIK3 protein in context of the invention has, preferably, an amino acid sequence shown in SIK3, Entry version 138 of 15-Mar-2017 or Entry version 144 of 28-Mar-2018. SIK3 is a cytoplasmatic protein with serine/threonine kinase activity which is regulated through phosphorylation of a conserved threonine residue (position 163) in the T-loop of the kinase domain by the LKB1 complex; a phosphorylation which is reported as essential for catalytic activity of SIK3 (Lizcano, J. M. et al.; EMBO J. 23, 833-843

(2004)). For the purposes of the herein disclosed invention the term "phosphorylated SIK3" shall denote a SIK3 protein that is phosphorylated substantially as SIK3 protein can be (eg is) phosphorylated by LKB1, wherein preferably such phosphorylated SIK3 comprising a phosphorthreonine at amino acid position 163. A phosphorylated SIK3 in context of the invention is an SIK3 protein that is activated in its cell-biological context. At least four protein isoforms (SIK3-001 to SIK3-004) generated by alternative splicing of the SIK3 gene product are known. The human SIK3 gene is located at chromosomal position 11q23.3 (HGNC gene Symbol Acc: HGNC:29165), and is conserved in many species such as in chimpanzee, Rhesus monkey, dog, cow, mouse, rat, chicken, zebrafish, and frog. The term SIK3 in some embodiments of the disclosure may also pertain to variants of the human SIK3 protein having an amino acid sequence that is substantially identical to, or of at least 80%, preferably 85%, more preferably 90, 95, 96, 97, 98, 99, or 100% sequence identity to, the amino acid sequence of SIK3 as described above, as determined using, e.g., the "Blast 2 sequences" algorithm described by Tatusova & Madden 1999 (FEMS Microbiol Lett 174: 247-250), and which (preferably) retain biological activity identical or substantially identical to the respective reference SIK3 (eg to phosphorylate one or more class II (eg IIa) HDACs, such as HDAC4). Preferred variants of SIK3 protein comprise sequence variants thereof due to sequence polymorphism between and within populations of the respective species, as well as mutations compared to the wild-type sequence of SIK3 which are located in or in close proximity to the activity loop or activation loop (T-loop) of SIK3. A preferred variant of SIK3 protein is a SIK3 T163 mutation, such as a mutation affecting the activation of SIK3. In preferred embodiments a SIK3 protein of the disclosure is not a SIK1 (synonyms: SIK and SNF1LK) protein and/or is not a SIK2 (synonyms: QIK, KIAA0781 and SNF1LK2) protein. The term SIK3 can mean, as applicable to the context (if not more specifically indicated), a SIK3 protein (such as one described above) or an mRNA molecule encoding such a SIK3 protein. The analogous meaning with respect of "SIK1" and "SIK2" is to be understood.

The compound of the invention is E9, which is the compound of formula (I), 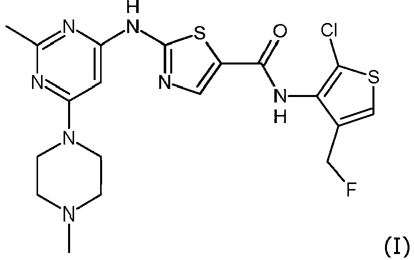 (*N*-(2-chloro-4-(fluoromethyl)thiophen-3-yl)-2-((2-methyl-6-(4-methylpiperazin-1-yl)pyrimidin-4-yl)amino)thiazole-5-carboxamide),
or a solvate or salt thereof.

In further disclosed aspects, derviatives of E9, such as any one of E1 to E8, or E10 to E16 as disclosed in WO2021/214117 (e.g. Figure 1 therein) can be used for the herein disclosed treatments or products. Methods for obtaining E9 or any of its derivatives are disclosed in WO2021/214117 or WO2023/067021.

The present invention also contemplates the following embodiments, which are further described elsewhere herein: The angiosarcoma may be at an advanced stage.

### Pharmaceutical compositions

In certain preferred embodiments, the amount of a compound in the pharmaceutical composition is an amount or dose sufficient for administration in an effective treatment regimen for treating angiosarcoma, in a subject.

The compounds and compositions described in the present invention or the compounds used in the present invention are preferably administered to a patient in need thereof via a pharmaceutical composition. Thus, in one aspect, the present disclosure provides a pharmaceutical composition comprising a kinase inhibitor as specified above under the heading "Compounds" (e.g., a kinase inhibitor having the general formula (I), and optionally one or more pharmaceutically acceptable excipients.

Thus, in one embodiment the pharmaceutical composition comprises a kinase inhibitor according to formula (I) as specified above under the heading "Compounds" (in particular a compound of the first aspect of the invention) and one or more pharmaceutically acceptable excipients. Furthermore, the pharmaceutical composition may further comprise one or more additional therapeutic agents. Thus, in particular embodiments, the pharmaceutical composition comprises (i) a kinase inhibitor according to formula (I) as specified above under the heading "Compounds" (in particular a compound of the first aspect of the invention) and one or more additional therapeutic agents; or (ii) a kinase inhibitor according to formula (I) as specified above under the heading "Compounds" (in particular a compound of the first aspect of the invention), one or more additional therapeutic agents, and one or more pharmaceutically acceptable excipients.

The term "pharmaceutically acceptable" refers to the non-toxicity of a material which does not interact with the (e.g., therapeutic) action of the active component (e.g., a kinase inhibitor of the invention (or a compound used in the invention), either alone or in combination with one or more additional therapeutic agents) of the pharmaceutical composition.

The pharmaceutical composition may be administered to an individual by any route. The pharmaceutical composition for use according to the invention is administered enterally or parenterally.

The expressions "enteral administration" and "administered enterally" as used herein mean that the drug administered is taken up by the stomach and/or the intestine. Examples of enteral administration include oral and rectal administration. The expressions "parenteral administration" and "administered parenterally" as used herein mean modes of administration other than enteral administration, usually by injection or topical application, and include, without limitation, intravenous, intramuscular, intraarterial, intrathecal, intracapsular, intraosseous, intraorbital, intracardiac, intradermal, intraperitoneal, transtracheal, subcutaneous, subcuticular, intraarticular, subcapsular, intracerebral, intracerebroventricular, subarachnoid, intraspinal, epidural and intrasternal administration (such as by injection and/or infusion) as well as topical administration (e.g., epicutaneous, inhalational, or through mucous membranes (such as buccal, sublingual or vaginal)).

Dosage forms for topical and/or transdermal administration of a compound described herein may include ointments, pastes, creams, lotions, gels, powders, solutions, sprays, inhalants, and/or patches. Generally, the active ingredient is admixed under sterile conditions with a pharmaceutically acceptable excipient such as one or more pharmaceutical carriers) and/or any needed preservatives and/or buffers as can be required. Additionally, the present disclosure contemplates the use of transdermal patches, which often have the added advantage of providing controlled delivery of an active ingredient to the body. Such dosage forms can be prepared, for example, by dissolving and/or dispensing the active ingredient in the proper medium. Alternatively, or additionally, the rate can be controlled by either providing a rate controlling membrane and/or by dispersing the active ingredient in a polymer matrix and/or gel.

Suitable devices for use in delivering intradermal pharmaceutical compositions described herein include short needle devices. Intradermal compositions can be administered by devices which limit the effective penetration length of a needle into the skin. Alternatively, or additionally, conventional syringes can be used in the classical mantoux method of intradermal administration. Jet injection devices which deliver liquid formulations to the dermis via a liquid jet injector and/or via a needle which pierces the stratum corneum and produces a jet which reaches the dermis are suitable. Ballistic powder/particle delivery devices which use compressed gas to accelerate the compound in powder form through the outer layers of the skin to the dermis are suitable.

Formulations suitable for topical administration include, but are not limited to, liquid and/or semi-liquid preparations such as liniments, lotions, oil-in-water and/or water-in-oil emulsions such as creams, ointments, and/or pastes, and/or solutions and/or suspensions. Topically administrable formulations may, for example, comprise from about 1% to about 10% (w/w) active ingredient, although the concentration of the active ingredient can be as high as the solubility limit of the active ingredient in the solvent. Formulations for topical administration may further comprise one or more of the additional ingredients described herein.

The compounds of the present invention (or the compounds used in the present invention) are generally applied in "pharmaceutically acceptable amounts" and in "pharmaceutically acceptable preparations". Such compositions may contain salts, buffers, preserving agents, carriers and optionally other therapeutic agents. "Pharmaceutically acceptable salts" comprise, for example, acid addition salts which may, for example, be formed by mixing a solution of compounds with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compound carries an acidic moiety, suitable pharmaceutically acceptable salts thereof may include alkali metal salts (e.g., sodium or potassium salts); alkaline earth metal salts (e.g., calcium or magnesium salts); and salts formed with suitable organic ligands (e.g., ammonium, quaternary ammonium and amine cations formed using counteranions such as halide, hydroxide, carboxylate, sulfate, phosphate, nitrate, alkyl sulfonate and aryl sulfonate). Illustrative examples of pharmaceutically acceptable salts include, but are not limited to, acetate, adipate, alginate, arginate, ascorbate, aspartate, benzenesulfonate, benzoate, bicarbonate, bisulfate, bitartrate, borate, bromide, butyrate, calcium edetate, camphorate, camphorsulfonate, camsylate, carbonate, chloride, citrate, clavulanate, cyclopentanepropionate, digluconate, dihydrochloride, dodecylsulfate, edetate, edisylate, estolate, esylate, ethanesulfonate, formate, fumarate, galactate, galacturonate, gluceptate, glucoheptonate, gluconate, glutamate, glycerophosphate, glycolylarsanilate, hemisulfate, heptanoate, hexanoate, hexylresorcinate, hydrabamine, hydrobromide, hydrochloride, hydroiodide, 2-hydroxy-ethanesulfonate, hydroxynaphthoate, iodide, isobutyrate, isothionate, lactate, lactobionate, laurate, lauryl sulfate, malate, maleate, malonate, mandelate, mesylate, methanesulfonate, methylsulfate, mucate, 2-naphthalenesulfonate, napsylate, nicotinate, nitrate, N-methylglucamine ammonium salt, oleate, oxalate, pamoate (embonate), palmitate, pantothenate, pectinate, persulfate, 3-phenylpropionate, phosphate/diphosphate, phthalate, picrate, pivalate, polygalacturonate, propionate, salicylate, stearate, sulfate, suberate, succinate, tannate, tartrate, teoclate, tosylate, triethiodide, undecanoate, valerate, and the like (see, for example, Berge et al., "Pharmaceutical Salts", J. Pharm. Sci., 66, pp. 1-19 (1977)).

The term "excipient" when used herein is intended to indicate all substances in a pharmaceutical composition which are not active ingredients (e.g., which are therapeutically inactive ingredients that do not exhibit any therapeutic effect in the amount/concentration used), such as, e.g., carriers, binders, lubricants, thickeners, surface active agents, preservatives, stabilizers, emulsifiers, buffers, flavoring agents, colorants, or antioxidants.

The compositions described in the present invention may comprise a pharmaceutically acceptable carrier. As used herein, "pharmaceutically acceptable carrier" includes any and all solvents, dispersion media, coatings, isotonic and absorption delaying agents, and the like that are physiologically compatible. The "pharmaceutically acceptable carrier" may be in the form of a solid, semisolid, liquid, or combinations thereof. Preferably, the carrier is suitable for enteral (such as oral) or parenteral administration (such as intravenous, intramuscular, subcutaneous, spinal or epidermal administration (e.g., by injection or infusion)); according to the invention, the administration is enteral or parenteral. Depending on the route of administration, the active compound, e.g., the compound of the present invention (or the compound used in the present invention), either alone or in combination with one or more additional therapeutic agents, may be coated in a material to protect the active compound(s) from the action of acids and other natural conditions that may inactivate the active compound.

Examples of suitable aqueous and non-aqueous carriers which may be employed in the pharmaceutical compositions according to the present invention include water (e.g., water for injection), ethanol, polyols (such as glycerol, propylene glycol, polyethylene glycol, and the like), aqueous solutions of a salt, carbohydrate, sugar alcohol, or an amino acid (such as saline or an aqueous amino acid solution), and suitable mixtures and/or buffered forms thereof, vegetable oils (such as olive oil), and injectable organic esters (such as ethyl oleate). Proper fluidity can be maintained, for example, by the use of coating materials, such as lecithin, by the maintenance of the required particle size in the case of dispersions, and by the use of surfactants.

Pharmaceutically acceptable carriers include sterile aqueous solutions or dispersions and sterile powders for the extemporaneous preparation of sterile injectable solutions or dispersions. The use of such media and agents for pharmaceutically active compounds is known in the art. Except insofar as any conventional media or agent is incompatible with the active compound, use thereof in the pharmaceutical compositions according to the present invention is contemplated.

The pharmaceutical composition described herein may comprise, in addition to the kinase inhibitor of the invention (and/or the compound or composition used in the invention), at least one, e.g., 1, 2, 3, 4, 5, 6, 7 or 8, additional therapeutic agents. According to the present teaching, the at least one additional therapeutic agent may be formulated together with the kinase inhibitor of the invention (and/or with the compound used in the invention) in a single pharmaceutical composition. Alternatively, the pharmaceutical composition may be structured as kit of parts, wherein the kinase inhibitor of the invention (or the compound used in the invention) is provided in a first formulation and the at least one additional therapeutic agent is provided in a second formulation, i.e., a second pharmaceutical composition. The first and the second pharmaceutical compositions may be combined prior to use. In other words, before administering the pharmaceutical composition, a formulation comprising the additional therapeutic agent may be added to the first pharmaceutical composition comprising the kinase inhibitor of the invention (or the compound used in the invention). Alternatively, the present teaching envisages administering the kinase inhibitor of the invention (or the compound used in the invention) formulated in a first pharmaceutical composition and administering the at least one additional therapeutic agent formulated in a second pharmaceutical composition. The pharmaceutical compositions may be administered concomitantly or in succession. For example, the first pharmaceutical composition may be administered at a first point in time and the second pharmaceutical composition may be administered at a second point in time, wherein the points in time may be separated by, for example, 0, or up to 1, 2, 3, 4, 5 or 10 min, up to 1, 2, 3, 4, 5 or 10 hours, up to 1, 2, 3, 4, 5 or 10 days, up to 1, 2, 3, 4, 5 or 10 weeks, up to 1, 2, 3, 4, 5 or 10 months or up to 1, 2, 3, 4, 5 or 10 years.

The compositions may also contain adjuvants such as preservatives, stabilizers, wetting agents, emulsifying agents, pH buffering agents, and dispersing agents. Prevention of the presence of microorganisms may be ensured by sterilization procedures and/or by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Regardless of the route of administration selected, the active compounds, which may be used in a suitable hydrated form, and/or the pharmaceutical compositions according to the present invention, are formulated into pharmaceutically acceptable dosage forms by conventional methods known to those of skill in the art (cf., e.g., Remington, "The Science and Practice of Pharmacy" edited by Allen, Loyd V., Jr., 22nd edition, Pharmaceutical Sciences, September 2012; Ansel et al., "Pharmaceutical Dosage Forms and Drug Delivery Systems", 7th edition, Lippincott Williams & Wilkins Publishers, 1999).

A pharmaceutical composition can be administered by a variety of methods known in the art. As will be appreciated by the skilled artisan, the route and/or mode of administration will vary depending upon the desired results. The pharmaceutical compositions containing one or more active compounds can be prepared with carriers that will protect the one or more active compounds against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Methods for the preparation of such compositions are generally known to those skilled in the art. See, e.g., Sustained and Controlled Release Drug Delivery Systems, J. R. Robinson, ed., Marcel Dekker, Inc., New York, 1978.

To administer a compound of the present invention (or a compound used in the present invention) by certain routes of administration, it may be necessary to coat the compound with, or co-administer the compound with, a material to prevent its inactivation. For example, the compound may be administered to an individual in an appropriate carrier, for example, liposomes, or a diluent. Pharmaceutically acceptable diluents include saline and aqueous buffer solutions. Liposomes include water-in-oil-in-water CGF emulsions as well as conventional liposomes (Strejan et al., J. Neuroimmunol. 7: 27(1984)).

Pharmaceutical compositions typically are sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

An injectable composition should be sterile and fluid to the extent that the composition is deliverable by syringe. In addition to water, the carrier can be an isotonic buffered saline solution, ethanol, polyol (for example, glycerol, propylene glycol, and liquid polyethylene glycol, and the like), and suitable mixtures thereof. Sterile injectable solutions can be prepared by incorporating the active compound in the required amount in an appropriate solvent with one or a combination of ingredients enumerated above, as required, followed by sterilization microfiltration.

Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the individuals to be treated; each unit contains a predetermined quantity of active compound calculated to produce the desired therapeutic effect in association with the required pharmaceutical carrier. The specification for the dosage unit forms used according to the present invention are dictated by and directly dependent on (a) the unique characteristics of the active compound and the particular therapeutic effect to be achieved, and (b) the limitations inherent in the art of compounding such an active compound for the treatment of sensitivity in individuals.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alphatocopherol, and the like; and (3) metal chelating agents, such as citric acid, ethylenediamine tetraacetic acid (EDTA), sorbitol, tartaric acid, phosphoric acid, and the like.

For the therapeutic/pharmaceutical formulations, compositions according to the present invention include those suitable for enteral administration (such as oral or rectal) or parenteral administration (such as nasal, topical (including vaginal, buccal and sublingual)). The compositions may conveniently be presented in unit dosage form and may be prepared by any methods known in the art of pharmacy. The amount of active ingredient (in particular, the amount of a compound according to the present invention) which can be combined with a carrier material to produce a pharmaceutical composition (such as a single dosage form) will vary depending upon the individual being treated, and the particular mode of administration. The amount of active ingredient which can be combined with a carrier material to produce a single dosage form will generally be that amount of the composition which produces a therapeutic effect.

Generally, out of 100% (for the pharmaceutical formulations/compositions), the amount of active ingredient (in particular, the amount of the compound according to the present invention (or of the compound used in the present invention), optionally together with other therapeutically active agents, if present in the pharmaceutical formulations/compositions) will range from about 0.01% to about 99%, preferably from about 0.1% to about 70%, most preferably from about 1% to about 30%, wherein the reminder is preferably composed of the one or more pharmaceutically acceptable excipients.

The amount of active ingredient, e.g., a compound according to the present invention (or a compound used in the present invention), in a unit dosage form and/or when administered to an individual or used in therapy, may range from about 0.1 mg to about 1000 mg (for example, from about 1 mg to about 500 mg, such as from about 10 mg to about 200 mg) per unit, administration or therapy. In certain embodiments, a suitable amount of such active ingredient may be calculated using the mass or body surface area of the individual, including amounts of between about 1 mg/kg and 10 mg/kg (such as between about 2 mg/kg and 5 mg/kg), or between about 1 mg/m² and about 400 mg/m² (such as between about 3 mg/m² and about 350 mg/m² or between about 10 mg/m² and about 200 mg/m²).

Actual dosage levels of the active ingredients in the pharmaceutical compositions according to the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A physician or veterinarian having ordinary skill in the art can readily determine and prescribe the (e.g., therapeutically) effective amount of the pharmaceutical composition required. For example, the physician or veterinarian could start with doses of the compounds according to the present invention (or of the compounds used in the present invention) at levels lower than that required in order to achieve the desired therapeutic effect and gradually increase the dosage until the desired effect is achieved. In general, a suitable daily dose of a composition according to the present invention will be that amount of the compound which is the lowest dose effective to produce a therapeutic effect. Such an effective dose will generally depend upon the factors described above. It is preferred that administration be oral, intravenous, intramuscular, intraperitoneal, or subcutaneous, preferably administered proximal to the site of the target. If desired, the (e.g., therapeutically) effective daily dose of a pharmaceutical composition may be administered as two, three, four, five, six or more sub-doses administered separately at appropriate intervals throughout the day, optionally, in unit dosage forms. While it is possible for a compound according to the present invention (or for the compound used in the present invention) to be administered alone, it is preferable to administer the compound as a pharmaceutical formulation/composition.

For oral administration, the pharmaceutical composition according to the present invention can take the form of, for example, tablets or capsules prepared by conventional means with pharmaceutical acceptable excipients such as binding agents (e.g., pregelatinised maize starch, polyvinylpyrrolidone, hydroxypropyl methylcellulose), fillers (e.g., lactose, microcrystalline cellulose, calcium hydrogen phosphate), lubricants (e.g., magnesium stearate, talc, silica), disintegrants (e.g., potato starch, sodium starch glycolate), or wetting agents (e.g., sodium lauryl sulphate). Liquid preparations for oral administration can be in the form of, for example, solutions, syrups, or suspensions, or can be presented as a dry product for constitution with water or other suitable vehicle before use. Such liquid preparation can be prepared by conventional means with pharmaceutically acceptable additives such as suspending agents (e.g., sorbitol, syrup, cellulose derivatives, hydrogenated edible fats), emulsifying agents (e.g., lecithin, acacia), non-aqueous vehicles (e.g., almond oil, oily esters, ethyl alcohol, fractionated vegetable oils), preservatives (e.g., methyl or propyl-p-hydroxycarbonates, sorbic acids). The preparations can also contain buffer salts, flavouring, coloring and sweetening agents as deemed appropriate. Preparations for oral administration can be suitably formulated to give controlled release of the pharmaceutical composition of the invention.

In one embodiment, the compound is orally administered in a concentration of, for example, at most 100 mg/kg body weight (such as at most 50 mg/kg body weight, at most 40 mg/kg body weight, at most 30 mg/kg body weight, at most 20 mg/kg body weight, at most 10 mg/kg body weight, at most 5 mg/kg body weight, at most 4 mg/kg body weight, at most 3 mg/kg body weight, at most 2 mg/kg body weight, at most 1 mg/kg body weight).

In one embodiment, the compound is parenterally administered (e.g., intravenously, intramuscularly, or subcutaneously), in a concentration of, for example, at most 10 mg/kg body weight (such as at most 5 mg/kg body weight, at most 4 mg/kg body weight, at most 3 mg/kg body weight, at most 2 mg/kg body weight, at most 1 mg/kg body weight, at most 0.5 mg/kg body weight, at most 0.4 mg/kg body weight, at most 0.3 mg/kg body weight, at most 0.2 mg/kg body weight, at most 0.1 mg/kg body weight).

The pharmaceutical composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc.

For administration by inhalation, the pharmaceutical composition according to the present invention is conveniently delivered in the form of an aerosol spray presentation from a pressurized pack or a nebulizer, with the use of a suitable propellant (e.g., dichlorodifluoromethane, trichlorofluoromethane, dichlorotetrafluoroethane, carbon dioxide, nitrogen, or other suitable gas). In the case of a pressurized aerosol, the dosage unit can be determined by providing a valve to deliver a metered amount. Capsules and cartridges of, for example, gelatine, for use in an inhaler or insufflator can be formulated containing a powder mix of the pharmaceutical composition according to the present invention and a suitable powder base such as lactose or starch.

The pharmaceutical composition according to the present invention can be formulated for parenteral administration by injection, for example, by bolus injection or continuous infusion. In one embodiment, the compounds or compositions according to the present invention may be administered by slow continuous infusion over a long period, such as more than 24 hours, in order to reduce toxic side effects. The administration may also be performed by continuous infusion over a period of from 2 to 24 hours, such as of from 2 to 12 hours. Such regimen may be repeated one or more times as necessary, for example, after 6 months or 12 months.

In yet another embodiment, the compounds or compositions according to the present invention are administered by maintenance therapy, such as, e.g., once a week for a period of 6 months or more.

Formulations for injection can be presented in units dosage form (e.g., in phial, in multidose container), and with an added preservative. The pharmaceutical composition according to the present invention can take such forms as suspensions, solutions or emulsions in oily or aqueous vehicles, and can contain formulatory agents such as suspending, stabilizing, or dispersing agents. Alternatively, the agent can be in powder form for constitution with a suitable vehicle (e.g., sterile pyrogen-free water) before use. Typically, compositions for intravenous administration are solutions in sterile isotonic aqueous buffer. Where necessary, the composition can also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients can be mixed prior to administration.

Compositions according to the present invention which are suitable for vaginal administration also include pessaries, tampons, creams, gels, pastes, foams or spray formulations containing such carriers as are known in the art to be appropriate. Dosage forms for the topical or transdermal administration of compositions according to the present invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

Therapeutic/pharmaceutical compositions can be administered with medical devices known in the art. For example, in a preferred embodiment, a therapeutic/pharmaceutical composition according to the present invention can be administered with a needleless hypodermic injection device, such as the devices disclosed in US 5,399,163; US 5,383,851; US 5,312,335; US 5,064,413; US 4,941,880; US 4,790,824; or US 4,596,556. Examples of well-known implants and modules useful in the present invention include those described in: US 4,487,603, which discloses an implantable micro-infusion pump for dispensing medication at a controlled rate; US 4,486,194, which discloses a therapeutic device for administering medicaments through the skin; US 4,447,233, which discloses a medication infusion pump for delivering medication at a precise infusion rate; US 4,447,224, which discloses a variable flow implantable infusion apparatus for continuous drug delivery; US 4,439,196, which discloses an osmotic drug delivery system having multi-chamber compartments; and US 4,475,916, which discloses an osmotic drug delivery system.

Many other such implants, delivery systems, and modules are known to those skilled in the art. In certain embodiments, the compounds according to the present invention can be formulated to ensure proper distribution *in vivo.* For example, the blood-brain barrier (BBB) excludes many highly hydrophilic compounds. To ensure that the compounds according to the present invention cross the BBB (if desired), they can be formulated, for example, in liposomes. For methods of manufacturing liposomes, see, e.g., US 4,522,811; US 5,374,548; and US 5,399,331. The liposomes may comprise one or more moieties which are selectively transported into specific cells or organs, and thus enhance targeted drug delivery (see, e.g., V.V. Ranade (1989) J. Clin. Pharmacol. 29: 685). Exemplary targeting moieties include folate or biotin (see, e.g., US 5,416,016 to Low et al.); mannosides (Umezawa et al., (1988) Biochem. Biophys. Res. Commun. 153: 1038); antibodies (P.G. Bloeman et al. (1995) FEBS Lett. 357: 140; M. Owais et al. (1995) Antimicrob. Agents Chemother. 39: 180); and surfactant protein A receptor (Briscoe et al. (1995) Am. J. Physiol. 1233: 134).

In one embodiment, the compounds according to the present invention (or the compounds used in the present invention) are formulated in liposomes. In a more preferred embodiment, the liposomes include a targeting moiety. In a most preferred embodiment, the compounds in the liposomes are delivered by bolus injection to a site proximal to the desired area. Such liposome-based composition should be fluid to the extent that easy syringability exists, should be stable under the conditions of manufacture and storage and should be preserved against the contaminating action of microorganisms such as bacteria and fungi.

A "therapeutically effective dosage" for therapy/treatment can be measured by objective responses which can either be complete or partial. A complete response (CR) is defined as no clinical, radiological or other evidence of a condition, disorder or disease. A partial response (PR) results from a reduction in disease of greater than 50%. Median time to progression is a measure that characterizes the durability of the objective tumor response.

A "therapeutically effective dosage" for therapy/treatment can also be measured by its ability to stabilize the progression of a condition, disorder or disease. The ability of a compound to inhibit one or more protein kinases or to reduce the viability of cells associated with a proliferative disorder, such as cancer cells can be evaluated by using appropriate *in-vitro* assays known to the skilled practitioner, such as those described herein (in particular in the Examples below). Alternatively, the properties of a compound described in the present invention can be evaluated by examining the ability of the compound in appropriate animal model systems known to the skilled practitioner such as those described herein (in particular in the Examples below). A therapeutically effective amount of a compound according to the present invention can cure, heal, alleviate, relieve, alter, remedy, ameliorate, improve or affect the condition, disorder or disease or the symptoms of the condition, disorder or disease or the predisposition toward the condition, disorder or disease in an individual. One of ordinary skill in the art would be able to determine such amounts based on such factors as the individual's size, the severity of the individual's symptoms, and the particular composition or route of administration selected.

The pharmaceutical composition according to the invention can also, if desired, be presented in a pack, or dispenser device which can contain one or more (e.g., unit) dosage forms containing the active compound. The pack can for example comprise metal or plastic foil, such as blister pack. The pack or dispenser device can be accompanied with a leaflet or other information; in particular, that describing (either to the patient and/or the administering physician) salient information or details on the pharmaceutical composition contained in the package, such as how to administer, recommended dosages, safety and/or side-effect information.

In a particular embodiment, a pharmaceutical composition of the invention is formulated for oral administration, and in an alternative particular embodiment, a pharmaceutical composition of the invention is formulated for intravenous administration.

In one embodiment, a pharmaceutical composition of the invention is in unit dose form, and in particular may be in a unit dose form that is formulated for oral administration.

Each of such a unit dose form may comprise (e.g., it may contain) between 1 and 950mg of the compound, such as the kinase kinase inhibitor of the first aspect or a compound used in the other aspects, complex, polymorph, crystalline form, racemic mixture, diastereomer, enantiomer, tautomer, conformer, isotopically labeled form, prodrug, or combination thereof. In particular, (e.g., each of) such a unit dose form may comprise (e.g., it may contain) between 2 and 150mg of such compound; and suitably, between 10 and 150mg of such compound.

In particular of such embodiments, a pharmaceutical composition of the invention that is in unit dose form (and in particular one be in a unit dose form that is formulated for oral administration) may comprise (e.g., it may contain) - for each unit dose form - about an amount of such compound selected from the list of amounts consisting of: 2mg, 5mg, 15mg, 20mg, 50mg, 70mg, 80mg, 100mg and 140mg; in particular, comprising (e.g., containing) an amount of about 20mg, 50mg, 70mg or 100mg of a compound of the invention (or of a compound used in the invention).

In one particular embodiment, the pharmaceutical composition of the invention is (e.g., is formed as) a tablet, caplet or capsule; suitably the pharmaceutical composition of the invention (e.g., a unit dose form thereof) is a caplet. Methods to form (e.g., manufacture) tablets and caplets are, for example, described elsewhere herein.

Suitable excipients for the pharmaceutical compositions of the invention, in particular when formed as a tablet or caplet, include, and particular embodiments of such a pharmaceutical composition of the invention include those that further comprise one or more (e.g., all of) the excipients selected from the list consisting of: lactose (e.g., lactose monohydrate), microcrystalline cellulose, croscarmellose sodium, hydroxypropylcellulose and magnesium stearate.

The kinase inhibitor described herein may be provided as a kit which comprises a first container and, optionally, a second containiner and a package insert. The first container contains at least one dose of a medicament comprising a kinase inhibitor as disclosed herein, the second container contains at least one dose of a medicament comprising a second therapeutic as described herein, and the package insert, or label, which comprises instructions for treating a patient for cancer using the medicaments. The first and second containers may be comprised of the same or different shape (e.g., vials, syringes and bottles) and/or material (e.g., plastic or glass). The kit may further comprise other materials that may be useful in administering the medicaments, such as diluents, filters, IV bags and lines, needles and syringes..

### Therapeutic and other applications

The present application provides a compound or composition as specified above, or a pharmaceutical composition as specified above under the heading "Pharmaceutical compositions" for use as a medicament, for example for use in therapy, in a coadministration, or combination therapy in a subject suffering from a soft tissue sarcoma or vascular tumor. The invention is as defined in the claims.

Treatment including or utilising such compounds may be provided at home, the doctor's office, a clinic, a hospital's outpatient department, or a hospital. Treatment generally begins under medical supervision so that medical personnel can observe the treatment's effects closely and make any adjustments that are needed. The duration of the treatment depends on the age and condition of the patient, as well as how the patient responds to the treatment.

A person having a greater risk of developing a condition, disorder or disease may receive prophylactic treatment to inhibit or delay symptoms of the condition, disorder or disease.

The term "treatment" is known to the person of ordinary skill, and includes the application or administration of a therapeutic agent (e.g., a pharmaceutical composition containing said agent) or procedure to a patient or application or administration of a therapeutic agent (e.g., a pharmaceutical composition containing said agent) or procedure to a cell, cell culture, cell line, sample, tissue or organ isolated from a patient, who has a condition, disorder or disease, a symptom of the condition, disorder or disease or a predisposition toward a condition, disorder or disease, with the purpose to cure, heal, alleviate, relieve, alter, remedy, ameliorate, improve, affect or prevent the condition, disorder or disease, the symptoms of the condition, disorder or disease or the predisposition toward the condition, disorder or disease. Hence, the term "treatment" can include prophylactic treatment of a condition, disorder or disease, or the symptom of a condition, disorder or disease. A therapeutic agent, when used in treatment, includes the kinase inhibitors of the invention (or the compounds used in the fifth aspect of the present invention) and includes, but is not limited to, additional therapeutic agents that may be small molecules, peptides, peptidomimetics, polypeptides/proteins, antibodies, nucleotides such as DNA or RNA, cells, viruses, ribozymes, siRNA, and antisense oligonucleotides.

The disease, disorder or a condition, in the context of the present description, is, in certain embodiments, a proliferative disorder (including a condition or symptom associated with such disorder).

A "proliferative disorder" refers to a disorder characterised by abnormal proliferation of cells. A proliferative disorder does not imply any limitation with respect to the rate of cell growth, but merely indicates loss of normal controls that affect growth and cell division. Thus, in some embodiments, cells of a proliferative disorder can have the same cell division rates as normal cells but do not respond to signals that limit such growth. Within the ambit of "proliferative disorder" is neoplasm or tumour, which is an abnormal growth of tissue or cells. Cancer is art understood, and includes any of various malignant neoplasms characterised by the proliferation of cells that have the capability to invade surrounding tissue and/or metastasise to new colonisation sites. Proliferative disorders include cancer, atherosclerosis, rheumatoid arthritis, idiopathic pulmonary fibrosis and cirrhosis of the liver. Non-cancerous proliferative disorders also include hyperproliferation of cells in the skin such as psoriasis and its varied clinical forms, Reiter's syndrome, pityriasis rubra pilaris, and hyperproliferative variants of disorders of keratinisation (e.g., actinic keratosis, senile keratosis), scleroderma, and the like.

According to the invention, the tumour is an angiosarcoma .

In reference to the various aspect disclosed above, the soft tissue sarcoma may be at an advanced stage.

In certain embodiments, a given patient having said disease can be resistant to a known therapy of soft tissue sarcoma or a vascular tumor. Such resistances may be against chemotherapeutic agents that are considered standard of case (paclitaxel, doxorubicin) or may be resistant to for example other targeted therapies such as anti-PD-1 therapies or anti-VEGFR/VEGFR therapies and may benefit from a therapy using E9 as specified herein. In such aspects it might be further advantegous to include such PD1-inhibitor (or anti VEGF/VEGFR agent) treatment along with the therapy of the invention.

### EXAMPLES

The examples show:

### Example 1: E9 single agent therapy with striking anti-tumor effect in a Angiosarcoma Xenograft (PDX) Model

The surprising E9 monotherapy efficacy was demonstrated in a Human Patient-Derived Xenograft (PDX) Angiosarcoma Model in Immuno-deficient Mice. This Angiosarcoma PDX model was used for preclinical evaluation of E9 in Angiosarcoma due to the retention of tumor heterogeneity and biological characteristics. Additionally, due to the rare indication, no murine syngeneic or human xenograft models are available.

Briefly, immuno-deficient NOD scid gamma (NOG) mice, were housed in a controlled environment conducive to tumor growth. Human PDX tumor samples, obtained from a patient with Angiosarcoma (Epitheloid AS of a female, 9 years old), were implanted subcutaneously into the mice. Tumor growth was monitored using non-invasive caliper method and was calculated by the following formula: volume = (width)2 × (length)/2.
Animals were randomized into treatment groups at a tumor volume (TV) of ~160mm3 (Table 1). Treatment started after randomization twice daily until termination at day 23 when Vehicle animals reached termination criteria due to TV.

**Table 1: Example treatment groups.**

| | Mice/group | Dose (mg/kg/dose) | Schedule |
|---|---|---|---|
| Vehicle Control * (5% w/v SBEβCD acidified with citric acid, pH 4.9-5) | 6 | - | p.o. / BID x 23 |
| E9 | 6 | 25 | p.o./ BID x 23 |
| E9 | 6 | 50 | p.o./ BID x 23 |

| | | | |
|---|---|---|---|
| *5% w/v SBEβCD acidified with citric acid, pH 4.9-5 p.o.: per os, oral administration via gavage BID: twice daily | | | |

Treatment with 25mg/kg E9 surprisingly already led to significant tumor growth inhibition (TGI) of 55%. The inventors were particularly surprised that treatment with 50 mg/kg of E9 even led to tumor regression, as tumor were smaller at termination compared to treatment start (see Figure 1A to 1D herein).

### Example 2 [prophetic]: E9 sensitivity in Angiosarcoma evaluated in In vitro assays with human derived cell lines

To prove E9 efficacy in Angiosarcoma and further strengthen the mechanism of action, human-derived Angiosarcoma cell lines are cultured in multi-well plates or microfluidic devices under controlled conditions of temperature, humidity, and nutrient supply. Cells are treated with test compounds, growth factors, or other stimuli to induce specific cellular responses.

Proliferation read-outs are determined using assays such as cell viability assays, cell counting methods, or automated imaging systems. Cell cycle analyses are performed using flow cytometry or immunofluorescence staining to quantify the distribution of cells in different phases of the cell cycle (G1, S, G2/M).

Functional kinase assays are conducted to assess the activity of specific kinases involved in signaling pathways relevant to cell growth, survival, and differentiation. This may involve the use of kinase inhibitors, substrates, or fluorescent probes followed by detection methods such as immunoblotting, enzyme-linked immunosorbent assay (ELISA), or others.

Western blot (WB) protein evaluation is employed to analyze changes in protein expression levels, post-translational modifications, or protein-protein interactions in response to experimental manipulations. Cell lysates are prepared, separated by gel electrophoresis, transferred to membranes, and probed with specific antibodies targeting proteins of interest. Protein bands are visualized using chemiluminescence or fluorescence detection methods.

### Example 3 [prophetic]: E9 efficacy in Angiosarcoma tested in Ex Vivo Organoid Assays

E9 efficacy in Angiosarcoma is further investigated in ex vivo organoid assays, including the 3D Collagen Tumor Growth (3DCTG) assay, and multi-dimensional analysis of organoid morphology, viability, and function.

Organoids, three-dimensional structures derived from stem cells or tissue explants, are cultured in extracellular matrix (ECM) substrates such as collagen, Matrigel, or synthetic hydrogels to mimic the native tissue microenvironment. The method allows for the expansion, differentiation, and manipulation of organoids derived from patient samples or cell lines representing various organs and diseases.

The 3DCTG assay involves embedding organoids in collagen matrices and monitoring their growth, invasion, and response to therapeutic agents. Time-lapse imaging techniques capture dynamic changes in organoid morphology and behavior over time, providing insights into cellular interactions and drug responses.

Multi-dimensional analysis of organoid assays encompasses the assessment of viability, proliferation, apoptosis, differentiation, and gene expression profiles using a combination of imaging, immunostaining, and molecular biology techniques. High-content imaging systems enable the quantitative analysis of organoid features such as size, shape, branching complexity, and fluorescence intensity.

### Example 4: Treatment of a secondary angiosarcoma in a female patient.

Clinical responder patient suffered from a secondary radiation-induced cutaneous angiosarcoma. The patient, who previously received radiotherapy in context of a treatment of breast cancer, developed secondary angiosarcoma. The angiosarcoma was treated with chemotherapy including paclitaxel, doxorubicin and cyclophosphamide the patient developed treatment resistance to the chemotherapy. In the following the patient received E9 in context of a dose excalation study in which E9 was administered orally every 12h (i.e. twice daily) for 28 days per treatment cycle. E9 daily doses were escalated from 10mg to 30mg and finally 60mg E9 during the then following continuous treatment. E9 dosing was well tolerated.

Surprisingly, the treatment with E9 resulted in significant and visible reduction of cutaneous leasions constituting a near-complete response,. Surpringly, further treatment with 60mg daily E9 maintained this near-complete response continuously over 10 months. The compound was well tolerated and the findings support applicability of E9 for the treatment of angiosarcoma.

## Claims

1. A **pharmaceutical composition for use in a treatment of angiosarcoma** in a subject, wherein the pharmaceutical composition is for enteral or parenteral administration to the subject and comprises a kinase inhibitor according to formula (I): 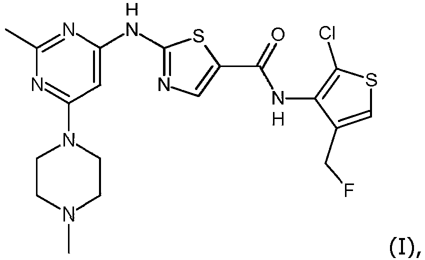 (*N-*(2-chloro-4-(fluoromethyl)thiophen-3-yl)-2-((2-methyl-6-(4-methylpiperazin-1-yl)pyrimidin-4-yl)amino)thiazole-5-carboxamide)
or solvates or salts thereof; together with a pharmaceutically acceptable carrier, diluent and/or excipient.

2. The pharmaceutical composition for use of claim 1, wherein the angiosarcoma is a primary or secondary angiosarcoma.

3. The pharmaceutical composition for use of claim 1 or 2, wherein the subject suffers from angiosarcoma and is **characterized by** a prior treatment history of radiotherapy for a different disease.

4. The pharmaceutical composition for use of claim 3, wherein the radiotherapy for a different disease is radiotherapy for the treatment of breast cancer.

5. The pharmaceutical composition for use of any one of claims 1 to 4, wherein the angiosarcoma is metastatic or unresectable.

6. The pharmaceutical composition for use of any one of claims 1 to 5, wherein the subject is resistant to chemotherapy.

7. The pharmaceutical composition for use of any one of claims 1 to 6, wherein the kinase inhibitor is formulated as a capsule or tablet.

8. The pharmaceutical composition for use of any one of claims 1 to 7, wherein the kinase inhibitor is formulated as a single dosage form comprising in one such single dosage form between 2mg and 150mg of the kinase inhibitor.

9. The pharmaceutical composition for use of any one of claims 1 to 8, wherein the kinase inhibitor is administered every 12 hours.

10. The pharmaceutical composition for use of any one of claims 1 to 9, wherein the treatment comprises an administration of at least one additional therapeutic to the subject, wherein the additional therapeutic is selected from radiotherapy, surgical removal of the tumor and administration of further therapeutic agents known to be effective for the treatment of angiosarcoma.

11. The pharmaceutical composition for use of claim 10, wherein the additional therapeutic is selected from an anti-PD1/PDL1 therapeutic, a taxane chemotherapeutic, an anthracycline, a PDGF/PDGFR inhibitor, a VEGF/VEGFR inhibitor, and β-blockers.

## Patentansprüche

1. **Pharmazeutische Zusammensetzung zur Verwendung bei einer Behandlung von Angiosarkom** bei einem Subjekt, wobei die pharmazeutische Zusammensetzung für eine enterale oder parenterale Verabreichung an das Subjekt bestimmt ist und einen Kinasehemmer gemäß Formel (I) umfasst: 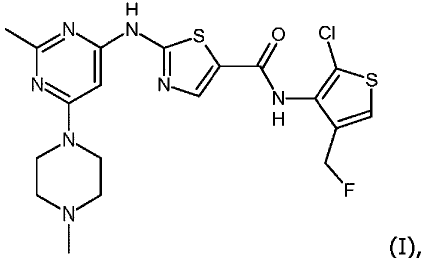 (*N*-(2-Chlor-4-(fluormethyl)thiophen-3-yl)-2-((2-methyl-6-(4-methylpiperazin-1-yl)pyrimidin-4-yl)amino)thiazol-5-carboxamid)
oder Solvate oder Salze davon; zusammen mit einem pharmazeutisch verträglichen Träger, Verdünner und/oder Hilfsstoff.

2. Pharmazeutische Zusammensetzung zur Verwendung nach Anspruch 1, wobei das Angiosarkom ein primäres oder sekundäres Angiosarkom ist.

3. Pharmazeutische Zusammensetzung zur Verwendung nach Anspruch 1 oder 2, wobei das Subjekt an Angiosarkom leidet und **durch** eine vorherige Behandlungsgeschichte einer Strahlentherapie für eine andere Krankheit **gekennzeichnet** ist.

4. Pharmazeutische Zusammensetzung zur Verwendung nach Anspruch 3, wobei die Strahlentherapie für eine andere Erkrankung eine Strahlentherapie für die Behandlung von Brustkrebs ist.

5. Pharmazeutische Zusammensetzung zur Verwendung nach einem der Ansprüche 1 bis 4, wobei das Angiosarkom metastasiert oder inoperabel ist.

6. Pharmazeutische Zusammensetzung zur Verwendung nach einem der Ansprüche 1 bis 5, wobei das Subjekt resistent gegen Chemotherapie ist.

7. Pharmazeutische Zusammensetzung zur Verwendung nach einem der Ansprüche 1 bis 6, wobei der Kinasehemmer als eine Kapsel oder eine Tablette formuliert ist.

8. Pharmazeutische Zusammensetzung zur Verwendung nach einem der Ansprüche 1 bis 7, wobei der Kinasehemmer als eine Einzeldosierungsform formuliert ist, umfassend in einer solchen Einzeldosierungsform zwischen 2 mg und 150 mg des Kinasehemmers.

9. Pharmazeutische Zusammensetzung zur Verwendung nach einem der Ansprüche 1 bis 8, wobei der Kinasehemmer alle 12 Stunden verabreicht wird.

10. Pharmazeutische Zusammensetzung zur Verwendung nach einem der Ansprüche 1 bis 9, wobei die Behandlung eine Verabreichung mindestens eines zusätzlichen Therapeutikums an das Subjekt umfasst, wobei das zusätzliche Therapeutikum aus Strahlentherapie, chirurgischer Entfernung des Tumors und Verabreichung weiterer therapeutischer Wirkstoffe, die bekanntermaßen für die Behandlung von Angiosarkom wirksam sind, ausgewählt ist.

11. Pharmazeutische Zusammensetzung zur Verwendung nach Anspruch 10, wobei das zusätzliche Therapeutikum aus einem Anti-PD1/PDL1-Therapeutikum, einem Taxan-Chemotherapeutikum, einem Anthrazyklin, einem PDGF/PDGFR-Hemmer, einem VEGF/VEGFR-Hemmer und β-Blockern ausgewählt ist.

## Revendications

1. **Composition pharmaceutique pour utilisation dans un traitement d'un angiosarcome** chez un sujet, dans laquelle la composition pharmaceutique est destinée à être administrée entérale ou parentérale au sujet et comprend un inhibiteur de kinase selon la formule (I) : 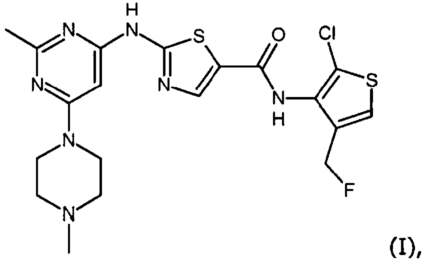 (*N*-(2-chloro-4-(fluorométhyl)thiophén-3-yl)-2-((2-méthyl-6-(4-méthylpipérazin-1-yle)pyrimidin-4-yl)amino)thiazole-5-carboxamide)
ou solvates ou sels de celui-ci ; conjointement avec un support, un diluant et/ou un excipient pharmaceutiquement acceptables.

2. Composition pharmaceutique pour utilisation selon la revendication 1, dans laquelle l'angiosarcome est un angiosarcome primaire ou secondaire.

3. Composition pharmaceutique pour utilisation selon la revendication 1 ou 2, dans laquelle le sujet souffre d'un angiosarcome et est **caractérisée par** des antécédents de traitement de radiothérapie pour une maladie différente.

4. Composition pharmaceutique pour utilisation selon la revendication 3, dans laquelle la radiothérapie pour une maladie différente est une radiothérapie pour le traitement du cancer du sein.

5. Composition pharmaceutique pour utilisation selon l'une quelconque des revendications 1 à 4, dans laquelle l'angiosarcome est métastatique ou non résécable..

6. Composition pharmaceutique pour utilisation selon l'une quelconque des revendications 1 à 5, dans laquelle le sujet est résistant à la chimiothérapie.

7. Composition pharmaceutique pour utilisation selon l'une quelconque des revendications 1 à 6, dans laquelle l'inhibiteur de kinase est formulé sous forme de gélule ou de comprimé.

8. Composition pharmaceutique pour utilisation selon l'une quelconque des revendications 1 à 7, dans laquelle l'inhibiteur de kinase est formulé sous une forme posologique unique comprenant dans une telle forme posologique unique entre 2 mg et 150 mg de l'inhibiteur de kinase.

9. Composition pharmaceutique pour utilisation selon l'une quelconque des revendications 1 à 8, dans laquelle l'inhibiteur de kinase est administré toutes les 12 heures.

10. Composition pharmaceutique pour utilisation selon l'une quelconque des revendications 1 à 9, dans laquelle le traitement comprend l'administration d'au moins un traitement supplémentaire au sujet, dans laquelle le traitement supplémentaire est choisi parmi la radiothérapie, l'ablation chirurgicale de la tumeur et l'administration d'autres agents thérapeutiques connus pour être efficaces pour le traitement de l'angiosarcome.

11. Composition pharmaceutique pour utilisation selon la revendication 10, dans laquelle le traitement supplémentaire est choisi parmi un agent thérapeutique anti-PD1/PDL1, un agent chimiothérapeutique taxane, une anthracycline, un inhibiteur de PDGF/PDGFR, un inhibiteur de VEGF/VEGFR et des β-bloquants.
